# EUROPEAN PATENT SPECIFICATION

(11) **EP 3 517 175 B1**
(45) Date of publication and mention of the grant of the patent: **17.03.2021**
(21) Application number: 19152993.2
(22) Date of filing: 22.01.2019
(51) Int. Cl.: A61P 27/06, A61K 36/05, A23L 17/00, A61P 27/02

(54) **COMPOSITION FOR IMPROVING EYE HEALTH COMPRISING TETRASELMIS**
ZUSAMMENSETZUNG ZUR VERBESSERUNG DER AUGENGESUNDHEIT MIT TETRASELMIS
COMPOSITION POUR AMÉLIORER LA SANTÉ OCULAIRE COMPRENANT DU TETRASELMIS

(30) Priority: 26.01.2018 KR 20180009841
(43) Date of publication of application: 31.07.2019
(73) Proprietor: Korea Institute of Science and Technology, Seoul 02792 (KR)
(72) Inventor: PAN, Cheol-Ho, 25451 Gangwon-do (KR); KANG, Kyungsu, 25451 Gangwon-do (KR); KIM, Sang Min, 02792 Seoul (KR); JUNG, Sang Hoon, 25451 Gangwon-do (KR); AHN, Hong Ryul, 25451 Gangwon-do (KR); EOM, Jae-In, 02792 Seoul (KR); KOO, Song Yi, 25451 Gangwon-do (KR); CHOI, Yongsoo, 25451 Gangwon-do (KR); YANG, Jung-Seok, 02792 Seoul (KR); LEE, Eun Ha, 25451 Gangwon-do (KR); LEE, Hee Ju, 25451 Gangwon-do (KR); KIM, Kyung A, 25451 Gangwon-do (KR)
(74) Representative: advotec.

(56) References cited:
- WO-A1-2016/177853
- AHMED FARUQ ET AL: "Profiling of carotenoids and antioxidant capacity of microalgae from subtropical coastal and brackish waters", FOOD CHEMISTRY, ELSEVIER LTD, NL, vol. 165, 27 May 2014 (2014-05-27), pages 300-306, XP029036421, ISSN: 0308-8146, DOI: 10.1016/J.FOODCHEM.2014.05.107
- GARCIA J L ET AL: "Microalgae, old sustainable food and fashion nutraceuticals", MICROBIAL BIOTECHNOLOGY 20170901 JOHN WILEY AND SONS LTD GBR, vol. 10, no. 5, 1 September 2017 (2017-09-01), pages 1017-1024, XP9513178, ISSN: 1751-7907
- DATABASE WPI Week 201552 Thomson Scientific, London, GB; AN 2015-347438 XP002791339, & KR 2015 0058715 A (KBR CO LTD) 29 May 2015 (2015-05-29)
- DATABASE GNPD [Online] MINTEL; February 2017 (2017-02), Anonymous: "Vital Greens Face Mist", XP002791340, Database accession no. 4593057

## Description

### BACKGROUND OF THE INVENTION

### Field of the Invention

The present disclosure discloses a composition for improving eye health comprising *Tetraselmis* sp. microalgae; being a fraction or an extract of the microalgae, as an active ingredient.

### Description of the Related Art

The retina is a nerve tissue that covers the innermost part of the eyeball. It is an organ that converts photic information into electrical information and transmits it to the brain. Retinopathy is a chronic or acute injury that occurs in the retina of the eye, and involves inflammation, vascular remodeling, etc. In addition, retinopathy may occur as an ocular manifestation of systemic diseases such as diabetes or hypertension. The retinopathy includes diabetic retinopathy, hypertensive retinopathy, and retinopathy of prematurity.

Marine microalgae are divided into green algae, brown algae, and red algae according to the depth and color of the sea. They contain pigments such as chlorophyll, carotenoid, and phycobilin and are classified as plants that grow and propagate mainly through photosynthesis and some of them are classified as bacteria in a broad sense. Marine microalgae have high biomass productivity and have an advantage that they can be easily grown in a natural environment allowing to obtain light energy as well as fresh water and seawater. Marine microalgae produce not only primary metabolites such as proteins, fats, and carbohydrates but also secondary metabolites produced from these metabolites and have very diverse biological activities. Thus, they are used in various fields including natural pigments, cosmetic raw materials, medicinal materials, biochemicals, feed, and alternative energy, in addition to health supplements AHMED FARUQ ET AL: FOOD CHEMISTRY, ELSEVIER LTD, NL, vol. 165, 27 May 2014, pp 300-306; GARCIA J L ET AL:, MICROBIAL BIOTECHNOLOGY JOHN WILEY AND SONS LTD GBR, vol. 10, no. 5, 1 September 2017, pp 1017-1024 each disclose that microalgae such as Tetraselmis contain carotenoids and that the carotenoids are associated with health benefits such as in macular degeneration.

### Citation List

### Patent Literature

Patent Literature 1: Korean Patent Laid-Open No. 10-2015-0035432

### SUMMARY OF THE INVENTION

The invention is as defined in the claims. In one aspect, the present disclosure is to provide a pharmaceutical composition for preventing or treating a retinal disease comprising *Tetraselmis* sp. microalgae as an active ingredient.

In another aspect, the present invention is to provide a health food composition for improving eye health comprising *Tetraselmis* sp. microalgae as an active ingredient.

In one aspect, the technology disclosed herein provides *Tetraselmis* sp. microalgae, as an extract of the microalgae, for improving eye health.

In one exemplary embodiment, the *Tetraselmis* sp. microalgae may be *Tetraselmis chuii.*

The extract is obtained by extracting *Tetraselmis* sp. microalgae, a culture thereof, a crushed material thereof using at least one solvent being hexane. The extract is hexane extract or a hexane fraction.

The improvement of eye health is the prevention, amelioration or treatment of a retinal disease.

In one exemplary embodiment, the retinal disease may be at least one selected from the group consisting of retinopathy of prematurity, retinal degeneration, aged macular degeneration, diabetic retinopathy, hypertensive retinopathy, retinitis pigmentosa, Leber congenital amaurosis, retinal detachment, glaucoma, and optic neuropathy.

In one exemplary embodiment, the retinal disease may be optic neuropathy caused by retinal optic nerve damage.

In one exemplary embodiment, the improvement of eye health may be the inhibition of retinal damage.

In one exemplary embodiment, the improvement of eye health may be the inhibition of retinal optic nerve damage.

In one exemplary embodiment, the *Tetraselmis* sp. microalgae, extract may inhibit retinal damage and improve the retinal function.

In one exemplary embodiment, the *Tetraselmis* sp. microalgae, extract may inhibit retinal optic nerve damage.

In another aspect, the technology disclosed herein provides non-therapeutic use of the *Tetraselmis* sp. microalgae, the culture thereof, the crushed material thereof, or the extract of the microalgae, the culture or the crushed material, for improving eye health.

In one aspect, the technology disclosed herein has the effect of providing a pharmaceutical composition for preventing or treating a retinal disease comprising *Tetraselmis* sp. microalgae as an active ingredient.

In another aspect, the technology disclosed herein has the effect of providing a health food composition for improving eye health comprising *Tetraselmis* sp. microalgae as an active ingredient.

### BRIEF DESCRIPTION OF THE DRAWINGS

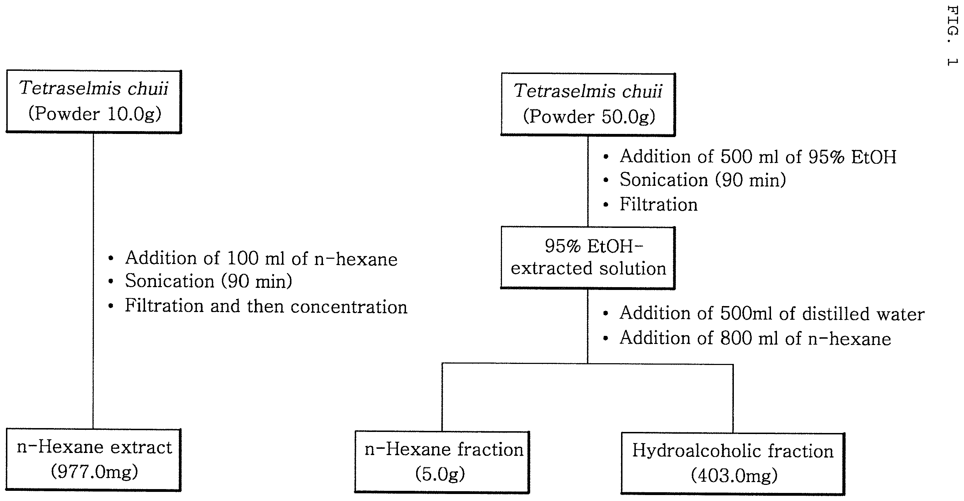

FIG. 1 is a schematic diagram showing a method of making a hexane extract and a hexane fraction from the microalgae used in the present disclosure, *Tetraselmis chuii.* Animal experiments were conducted using the extract and fraction of *Tetraselmis chuii* produced by the method, in order to verify their optic nerve protective effect;

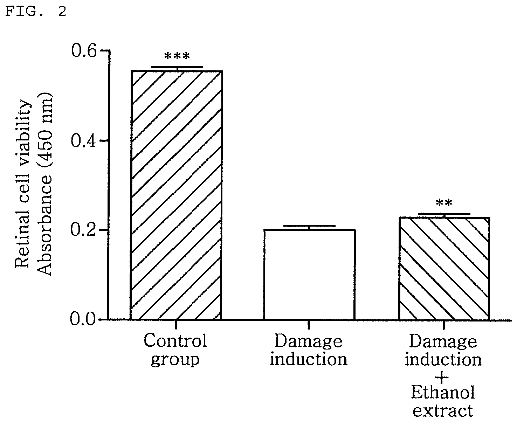

FIG. 2 is a graph showing the retinal cell viability recovered by the administration of 50 µg/mL of an ethanol extract of *Tetraselmis chuii* after the induction of retinal damage in retinal precursor cells R28 according to a test example of the present disclosure. A statistical significance difference compared with a test group under retinal damage-inducing conditions based on statistical analysis is also indicated. The statistical significances were ***P* < 0.01 and ****P* < 0.001.

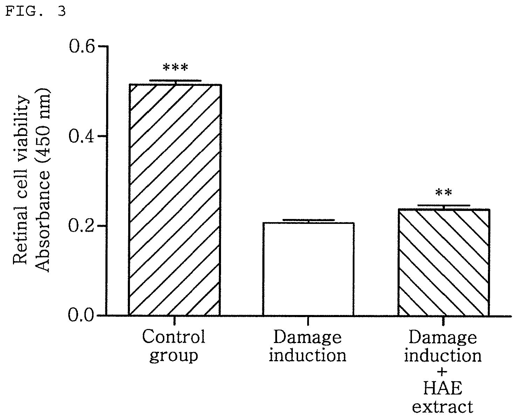

FIG. 3 is a graph showing the retinal cell viability recovered by the administration of 50 µg/mL of the HAE extract (extracted with a mixed solvent of n-hexane, acetone, and ethanol at a ratio of 2: 1: 1) of *Tetraselmis chuii* after the induction of retinal damage in retinal precursor cells R28 according to a test example of the present disclosure. A statistical significance difference compared with a test group under retinal damage-inducing conditions based on statistical analysis is also indicated. The statistical significances were ***P* < 0.01 and ****P* < 0.001.

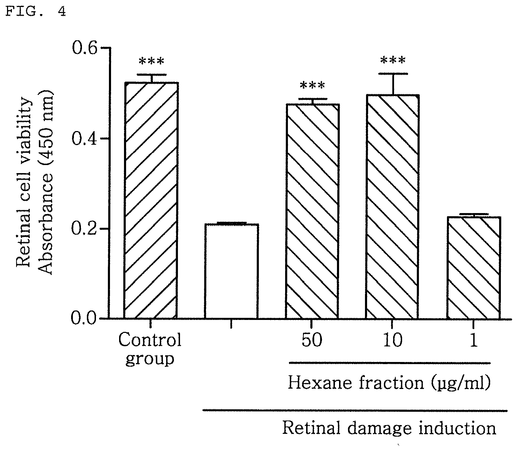

FIG. 4 is a graph showing the retinal cell viability recovered by the administration of 1, 10 and 50 µg/mL of the hexane fraction of *Tetraselmis chuii* after the induction of retinal damage in retinal precursor cells R28 according to a test example of the present disclosure. A statistical significance difference compared with a test group under retinal damage-inducing conditions based on statistical analysis is also indicated. The statistical significance was ****P* < 0.001.

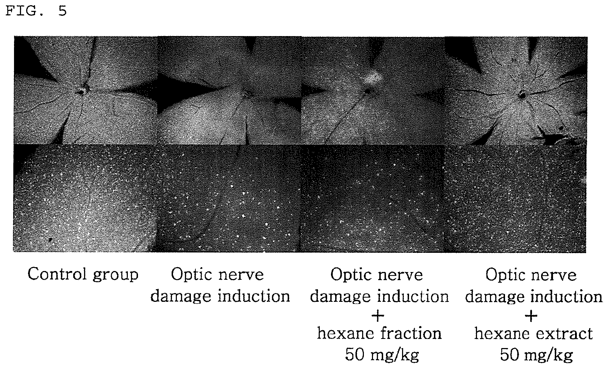

FIG. 5 is a photomicrograph of retinal tissues showing the optic nerve damage inhibitory effect achieved by the administration of a hexane fraction of *Tetraselmis chuii* (50 mg/kg) and a hexane extract of *Tetraselmis chuii* (50 mg/kg) in an optic nerve crush model in mouse according to a test example of the present disclosure.

### DETAILED DESCRIPTION OF THE PREFERRED EMBODIMENTS

Hereinafter, the present invention will be described in detail.

In one aspect, the technology disclosed herein provides a pharmaceutical composition for preventing or treating a retinal disease comprising *Tetraselmis* sp. microalgae, a culture thereof, a crushed material thereof, or an extract of the microalgae, the culture or the crushed material as an active ingredient.

In another aspect, the technology disclosed herein provides a health food composition for improving eye health comprising *Tetraselmis* sp. microalgae, a culture thereof, a crushed material thereof, or an extract of the microalgae, the culture or the crushed material as an active ingredient.

In another aspect, the technology disclosed herein provides *Tetraselmis* sp. microalgae, a culture thereof, a crushed material thereof, or an extract of the microalgae, the culture or the crushed material for improving eye health.

In another aspect, the technology disclosed herein provides a non-therapeutic use of *Tetraselmis* sp. microalgae, a culture thereof, a crushed material thereof, or an extract of the microalgae, the culture or the crushed material, for improving eye health.

In one exemplary embodiment, the *Tetraselmis* sp. microalgae, a culture thereof, a crushed material thereof, or an extract of the microalgae, the culture or the crushed material may be applied or administered to a subject in the form of a composition, for example, a pharmaceutical composition or a food composition.

As used herein, the term "active ingredient" refers to an ingredient that exhibits the desired activity by itself or an ingredient which can exhibit the desired activity together with a carrier which itself has no activity, etc.

*Tetraselmis* sp. microalgae are unicellular or colonial microalgae which include chlorophyll a and b as photosynthetic pigments and produce mannitol as an initial photosynthetic product and which contain galactose, uronic acid, etc. as the main components of the cell wall.

In one exemplary embodiment, the *Tetraselmis* sp. microalgae may be *Tetraselmis chuii.*

As used herein, the active ingredient has a broad meaning covering not only *Tetraselmis* sp. microalgae, a culture thereof, a crushed material thereof, or an extract of the microalgae, the culture or the crushed material, but also a processed article thereof, for example, any material that can be obtained by further processing or treating the obtained product by drying, concentration, fractionation, fermentation, etc.

In one exemplary embodiment of the disclosure , the culture may refer to a material containing some or all of the materials contained in a medium in which microalgae have been cultured, regardless of the form of the culture. For example, it may refer to a material containing a metabolite or secretion resulting from microalgae culture or a crushed material thereof. The culture may also contain microalgae themselves.

In one exemplary embodiment, the crushed material may refer to a product obtained by crushing microalgae themselves by a chemical or physical means.

In one exemplary embodiment, the extract may be a product obtained by extracting or fractionating the microalgae, culture, or crushed material, regardless of the extraction method, the extraction solvent, the extracted component or the form of the extract.

In one exemplary embodiment, the extract may be an extract prepared according to a conventional extraction method used in the art. For example, the extract may be extracted using a solvent extraction method using at least one solvent selected from the group consisting of water, anhydrous or hydrated alcohol having 1 to 6 carbon atoms, for example, methanol, ethanol, propanol or butanol, propylene glycol, butylene glycol, dipropylene glycol, glycerin, acetone, ethyl acetate, chloroform, methylene chloride, butyl acetate, diethyl ether, dichloromethane (not claimed) and hexane.

In one exemplary embodiment, the method for preparing the extract may be a conventional extraction method known in the art, including a method using an extraction device such as supercritical extraction, subcritical extraction, high temperature extraction, high pressure extraction, or ultrasonic extraction, or a method using an adsorption resin including XAD and HP-20. Specifically, the extraction may be heat reflux extraction or room temperature extraction, although not limited thereto. In addition, the extraction may be performed 1 to 5 times, and specifically, the extraction may be repeated 3 times, although not limited thereto. The extraction time may be 2 to 100 hours, specifically 10 to 70 hours, although not limited thereto.

According to the invention the extract is hexane extract.

In one exemplary embodiment, the extract may be a hexane fraction, specifically a hexane fraction of an ethanol extract.

In one exemplary embodiment, the content of the active ingredient may be 0.001 to 10% by weight based on the total weight of the composition.

In one exemplary embodiment, an extract of the microalgae, may prevent, ameliorate, alleviate, or treat a retinal disease.

In one exemplary embodiment, the extract of the microalgae, may inhibit retinal damage and improve the retinal function.

In one exemplary embodiment, the extract of the microalgae, may inhibit the retinal damage caused by oxidative stress.

In one exemplary embodiment, the extract of the microalgae, according to the present disclosure may inhibit retinal optic nerve damage and thereby improve eye health.

In one exemplary embodiment, the retinal disease may be at least one selected from the group consisting of retinopathy of prematurity, retinal degeneration, aged macular degeneration, diabetic retinopathy, hypertensive retinopathy, retinitis pigmentosa, Leber congenital amaurosis, retinal detachment, glaucoma, and optic neuropathy.

In one exemplary embodiment, the optic neuropathy may include optic neuritis, genetic optic neuropathy, metabolic optic neuropathy, optic neuropathy due to a toxic agent, and optic neuropathy caused by adverse drug effects or vitamin deficiency.

In one exemplary embodiment, the retinal disease may be a degenerative retinal disease.

In one exemplary embodiment, the retinal disease may be caused by oxidative stress.

In one exemplary embodiment, the retinal disease may be intraretinal neuropathy caused by retinal optic nerve damage.

The pharmaceutical compositions of the present invention may further comprise a suitable carrier, excipient, and diluent conventionally used in the preparation of medicaments.

Examples of the carrier, excipient, and diluent that can be included in the pharmaceutical composition of the present invention include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methylhydroxybenzoate, propylhydroxybenzoate, talc, magnesium stearate, and mineral oil.

The pharmaceutical composition according to the present invention may be formulated into oral formulations such as powders, granules, tablets, capsules, suspensions, emulsions, syrups and aerosols, external medicines, suppositories and sterile injection solutions.

When the composition is formulated, generally used diluents or excipients such as fillers, extenders, binders, humectants, disintegrants, and surfactants are used. Solid preparations for oral administration include tablets, pills, powders, granules, and capsules, and are prepared by mixing the composition of the present invention with at least one excipient, for example, starch, calcium carbonate, sucrose or lactose, gelatin, etc. Further, lubricants such as magnesium stearate, talc, etc. are used in addition to simple excipients. Liquid preparations for oral administration include suspensions, liquids for internal use, emulsions, syrups, etc., and various excipients such as humectants, sweeteners, flavoring agents, preservatives, etc. as well as water and liquid paraffin, which are generally used simple diluents, may be added. Formulations for parenteral administration include sterile aqueous solutions, nonaqueous solvents, suspensions, emulsions, freeze-dried formulations, and suppositories. Examples of the nonaqueous solvents and the suspension solvents include propylene glycol, polyethylene glycol, vegetable oil such as olive oil, and injectable esters such as ethyl oleate. Examples of the matrix for suppositories include witepsol, macrogol, tween 61, cacao butter, laurinum, and glycerogelatin.

The composition of the present invention may be administered orally or parenterally (for example, intravenous, subcutaneous, or intraperitoneal or local application) according to the desired method. The dosage may vary depending on the patient's condition, body weight, age, gender, diet, excretion rate, severity of disease, form of drug, time of administration, administration method, administration route, duration of administration etc. The daily dose of the freeze-dried active ingredient according to the present invention is 0.0001 mg/kg to 500 mg/kg, preferably 0.001 mg/kg to 100 mg/kg. It may be administered once a day or in several divided doses per day according to the need.

The health food composition of the present invention may be in the form of drinks, meats, sausages, bread, biscuits, rice cakes, chocolate, candies, snacks, confectionery, pizzas, ramen, other noodles, gums, dairy products including ice creams, various soups, beverages, alcoholic beverages, multi-vitamin preparations, etc. The health food encompasses all health foods in a conventional sense.

The active ingredient of the present invention may be added directly to a food or used together with other foods or food ingredients, and may be appropriately used according to a conventional method. The amount of the active ingredient to be mixed may be appropriately determined according to the intended use (for prevention or amelioration). In general, the content of the active ingredient in a health functional food may be 0.1 to 90 parts by weight of the total weight of the food. However, in the case of long-term intake for the purpose of health and hygiene or for the purpose of controlling health conditions, the amount may be less than the above range. Also, the active ingredient may be used in an amount exceeding the above range since it has no problem with stability.

The health functional beverage composition according to the present invention has no particular limitation on other ingredients, except that the active ingredient at a predetermined ratio is included as an essential ingredient. It may comprise various flavoring agents, natural carbohydrates or the like as additional ingredients, as do common beverages. Examples of the natural carbohydrates include conventional sugars such as monosaccharides, for example, glucose and fructose; disaccharides, for example, maltose and sucrose; and polysaccharides, for example, dextrin and cyclodextrin, and sugar alcohols such as xylitol, sorbitol, and erythritol. Also, natural flavoring agents (thaumatin, stevia extract (for example, rebaudioside A, glycyrrhizin, etc.)) and synthetic flavoring agents (for example, saccharin, aspartame, etc.) may be used as the flavoring agents. In general, the natural carbohydrate may be included in an amount of about 1 to 20 g, preferably about 5 to 12 g per 100 ml of the composition of the present invention.

In addition to the above, the active ingredient of the present invention may contain various nutrients, vitamins, minerals (electrolytes), flavors such as synthetic flavors and natural flavors, coloring agents, enhancers (cheese, chocolate, etc.), pectic acid and its salts, alginic acid and its salts, organic acids, protective colloidal thickeners, pH regulators, stabilizers, antiseptics, glycerin, alcohols, and carbonators used for carbonated drinks, etc. The active ingredient of the present invention may also contain natural fruit juice and fruit flesh for preparation of fruit beverages and vegetable beverages. These ingredients may be used alone or as a mixture. The content of these additives is generally about 0.1 to 20 parts by weight based on 100 parts by weight of the active ingredient of the present invention.

Hereinafter, the present invention will be described in more detail with reference to examples. It will be apparent to those skilled in the art that these examples are for illustrative purposes only, and the scope of the present invention is not construed as being limited by these examples.

### Example 1: Culture of Tetraselmis chuii

The microalgae used in this example, *Tetraselmis chuii,* was purchased from UTEX, USA. The microalgae number was UTEX LB 232.

Mass culture of *Tetraselmis chuii* was performed using F/2 medium made of sterilized seawater (see Table 1 below). In general, the culture fluid used in a mass culture of microalgae is chemically sterilized using 12% sodium hypochlorite (NaOCl), instead of autoclaving. For sterilization, seawater was treated with 1 mL of 12% NaOCl per L of seawater and left at room temperature for 24 hours. After 24 hours, in order to neutralize the remaining chlorine, sodium thiosulfate (Na₂S₂O₃) was used at a concentration of 120 mg/mL of 12% NaOCl to remove chlorine. Then the cells were inoculated at an initial concentration of 1.0×10⁵ cells/mL and cultured under natural light conditions at 20±2°C with an air supply through an air pump. After coagulation of the culture fluid of *Tetraselmis chuii,* centrifugation was performed to collect biomass, followed by freeze drying to obtain a dried powder.

**Table 1**

| Components | Contents (mg/L) |
|---|---|
| NaNO₃ | 75 |
| NaH₂PO₄·H₂O | 5 |
| NaSiO₃·9H₂O | 30 |
| CuSO₄·H₂O | 0.0098 |
| Na₂MoO₄·2H₂O | 0.0063 |
| ZnSO₄·7H₂O | 0.022 |
| MnCl₂·4H₂O | 0.18 |
| CoCl₂·6H₂O | 0.01 |
| FeCl₃·6H₂O | 3.15 |
| Na₂EDTA· 2H₂O | 4.36 |
| Cyanobalamin | 0.001 |
| Thiamine-HCl | 0.2 |
| Biotin | 0.001 |

### Example 2: Preparation of an extract and fraction of Tetraselmis chuii for testing their cell protective effect on retinal precursor cells

300 mL of 95% ethanol was added to 30 g of powders of dried *Tetraselmis chuii* and the mixture was stirred in a dark place at room temperature for 24 hours to carry out extraction. The resultant was filtered through filter paper, and 150 mL of the resultant extracted solution was concentrated under reduced pressure to obtain 1.1709 g of an ethanol extract.

In addition, 150 mL of distilled water was added to 150 mL of the obtained extracted solution remaining after preparation of the ethanol extract, and the resultant mixture was added with 200 mL of n-hexane. The resultant solution was mixed well, and after layer separation, the supernatant was separated. The fractionation procedure was repeated 3 times, and then the solvent was concentrated under reduced pressure to obtain 312.8 mg of a n-hexane fraction.

In addition, an HAE extract of *Tetraselmis chuii* (extracted with a mixed solvent of n-hexane, acetone, and ethanol at a ratio of 2: 1: 1) was prepared to contain a high content of carotenoids. It was prepared in the following manner: 100 mL of a mixed solvent of n-hexane, acetone, and ethanol at a ratio of 2: 1: 1 was added to 10 g of dried *Tetraselmis chuii* powders, and the mixture was stirred in a dark place at room temperature for 24 hours to carry out extraction. The extracted solution obtained by filtering the resultant through filter paper was concentrated under reduced pressure to obtain 726.8 g of an HAE extract of *Tetraselmis chuii,* which is an extract containing a high content of carotenoids.

### Example 3: Preparation of a hexane extract and hexane fraction of Tetraselmis chuii to be used for a test to identify their optic nerve protective effect in an optic nerve crush animal model

100 g of n-hexane was added to 10 g of dried *Tetraselmis chuii* powders, and ultrasonic extraction was performed in a dark place for 90 minutes. The extracted solution obtained by filtering the resultant through filter paper was concentrated under reduced pressure to obtain 977.0 mg of a hexane extract of *Tetraselmis chuii.*

Also, in order to prepare a hexane fraction of *Tetraselmis chuii,* 500 mL of 95% ethanol was added to 50 g of dried *Tetraselmis chuii* powders, and ultrasonic extraction was performed in a dark place for 90 minutes. 500 mL of distilled water was added to the extracted solution obtained by filtering the resultant through filter paper, and 800 mL of n-hexane was further added thereto. The resultant solution was mixed well, and after layer separation, the supernatant was separated. The fractionation procedure was repeated 3 times, and then the solvent was concentrated under reduced pressure to obtain 5.0 g of a hexane fraction of *Tetraselmis chuii.* FIG. 1 shows the method for preparation of the hexane extract and hexane fraction of *Tetraselmis chuii.*

### Test Example 1: Test of the cell protective effect on retinal precursor cells R28

The retinal precursor cell R28 was cultured in a constant temperature incubator at 37°C and 5% carbon dioxide/95% air under saturated humidity using Dulbecco's modified Eagle's medium (DMEM, Hyclone, USA) containing 10% fetal bovine serum (FBS) and 100 U/mL of penicillin/streptomycin. After 2 to 3 days, cells grown to saturation were removed from the culture flask using a trypsin solution and then subcultured. The cells were seeded in a 96-well culture plate and cultured in a constant temperature incubator for 24 hours to attach the cells. The medium was then removed and replaced with DMEM medium containing 1% low-concentration fetal bovine serum (FBS). The ethanol extract, HAE extract, and hexane fraction of *Tetraselmis chuii* according to Example 2 were pretreated to final concentrations of 50, 10, and 1 µg/mL 1 hour before application of oxidative stress. Then, 100 µL of each of L-buthionine-sulfoximine and L-glutamic acid was added to the 96-well culture plate to final concentrations of 0.5 mM L-buthionine-sulfoximine and 10 mM L-glutamic acid, followed by culture at 37°C for 24 hours. After completion of culture, the cell viability was measured using Ez-cytox (Daeil Lab Service, Korea) kit. Ez-cytox solution was diluted 10-fold with serum-free medium, and 100 µL of the resultant was added to each of 96 wells, followed by culture for 2 hours. After 2 hours, the absorbance was measured at 450 nm with a microplate reader. The cell viability is proportional to the absorbance.

The results showed that when stress was induced by treatment with 0.5 mM L-buthionine-sulfoximine and 10 mM L-glutamic acid to induce retinal damage in retinal precursor cells (R28), the retinal cell viability significantly decreased compared to that of the control group. In contrast, when the ethanol extract of *Tetraselmis chuii* (FIG. 2), the HAE extract of *Tetraselmis chuii* (FIG. 3) and the hexane fraction of *Tetraselmis chuii* (FIG. 4) were applied, the retinal cell viability was significantly recovered compared with that of the test group in which retinal damage was induced by oxidative stress.

Thus, it was found that the extract and fraction of *Tetraselmis chuii* can protect retinal precursor cells R28 from the retinal damage induced by oxidative stress.

### Test Example 2: Test of the retinal optic nerve protective effect of an extract and fraction of Tetraselmis chuii in C57BL/6J mice whose optic nerve has been damaged by an optic nerve crush model

Animal experiments were carried out to identify the optic nerve protective effect of the hexane extract and hexane fraction of *Tetraselmis chuii* prepared in Example 3. Optic nerve crush was used to induce the death of the retinal optic nerve in C57BL/6J mice. At this time, the extract and fraction of *Tetraselmis chuii* were administered to identify their effect in inhibiting optic nerve cell death.

Specifically, two days before the operation for inducing optic nerve damage, C57BL/6J mice were orally administered with 50 mg/kg of the hexane extract and hexane fraction of *Tetraselmis chuii.* At this time, the control group was orally administered with physiological phosphate buffer. To induce optic nerve damage, 2% Rompun injection and Zoletil were mixed and injected at 1 mL/kg intramuscularly to anesthetize the mouse. The back of the conjunctiva was carefully incised so as not to injure the blood vessels, to expose the optic nerve. Using cross-action forceps, the optic nerve 2 mm behind the eyeball was crushed for 5 seconds. 1 drop of Vigamox 0.5% eye drops (Alcon Laboratories) was applied to prevent postoperative infection. For the next 12 days, the test group was orally administered once a day with 50 mg/kg of the extract and fraction of *Tetraselmis chuii,* and the control group was orally administered with the same dose of physiological saline. After 6 days of optic nerve damage, the mouse was anesthetized again and the scalp was incised. In order to stain the optic nerve cells, the upper part of the brain connected to the optic nerve was perforated using a surgical drill and 3% FluoroGold was injected thereto. The incision was sutured and antibiotic ointment was applied to prevent infection. 12 days after optic nerve damage, the eyeball was removed and fixed in 4% parafromaldehyde for 4 hours. The retina was separated from the choroid of the fixed eyeball and incised into four radial sections, which were then mounted on a slide in the shape of a cloverleaf. Fluorescent optic nerve cells on the slide of each test group were identified and photographed at a magnification of 40 times and 100 times using a fluorescence microscope.

The results showed that optic nerve-crushed mice have clearly damaged optic nerve. In contrast, mice orally administered with a hexane extract and fraction of *Tetraselmis chuii* showed significantly reduced optic nerve damage. In particular, the hexane extract was found to preserve the optic nerve to an extent similar to that of the control group (FIG. 5).

Thus, the extract and fraction of *Tetraselmis chuii* according to the present disclosure were found to have an optic nerve protective effect in not only in vitro test but also optic nerve crush animal model. Thus, it was found that the extract and fraction of *Tetraselmis chuii* according to the present disclosure can be effectively used as a pharmaceutical composition and health food composition for preventing degenerative retinal diseases.

Hereinafter, formulation examples of the composition according to one aspect of the present disclosure will be described. However, it may be formulated into various other forms. These examples are for illustrative purposes only and are not intended to limit the scope of the present invention.

### [Formulation Example 1] Preparation of ophthalmic gel

5 mg of *Tetraselmis chuii* extract
20 mg of Carbopol 934
q.s. triethanolamine
2 mg of methyl p-oxybenzoate
Sterile purified water ad. 1 g
Methyl p-oxybenzoate was added to sterile purified water, and the mixture was heated for dissolution and then cooled to dissolve the *Tetraselmis chuii* extract. Carbopol 934 was added thereto, and the mixture was mixed for dispersion using a high-speed stirrer and then allowed to stand to remove air. Triethanolamine was added dropwise thereto with care to prevent air entry, thus preparing an ophthalmic gel.

### [Formulation Example 2] Preparation of ophthalmic solution

5 mg of *Tetraselmis chuii* extract
0.1 g of benzalkonium chloride
5 g of sodium chloride
6.2 g of boric acid
1.0 g of tyloxapol
q.s. dilute hydrochloric acid
Sterile purified water ad. 1000 ml
Sodium chloride and boric acid were added to *Tetraselmis chuii* extract in order and dissolved. Then, the mixture was added with benzalkonium chloride and tyloxapol dissolved in a small amount of sterile purified water, followed by stirring. The pH was adjusted by adding dilute hydrochloric acid. Sterilization was performed using a 0.45 microfilter.

### [Formulation Example 3] Preparation of ophthalmic ointment

5 mg of *Tetraselmis chuii* extract
10 mg of sterile purified water
2 mg of methyl p-oxybenzoate
100 mg of anhydrous lanolin
White Vaseline ad. 1 g
Methyl p-oxybenzoate was added to a sterilized glass bowl and *Tetraselmis chuii* extract was added thereto and dissolved. The mixture was mixed with anhydrous lanolin and mixed until homogeneous, thus preparing an ophthalmic ointment.

### [Formulation Example 4] Soft capsule

150 mg of *Tetraselmis chuii* extract, 2 mg of palm oil, 8 mg of hydrogenated palm oil, 4 mg of yellow wax, and 6 mg of lecithin were mixed. 400 mg of the resultant mixture was filled in a capsule according to a conventional method to prepare a soft capsule.

### [Formulation Example 5] Tablet

150 mg of *Tetraselmis chuii* extract, 100 mg of glucose, 50 mg of red ginseng extract, 96 mg of starch, and 4 mg of magnesium stearate were mixed and added with 40 mg of 30% ethanol to form granules. The granules were dried at 60°C and tabletted using a tablet machine.

### [Formulation Example 6] Granule

150 mg of *Tetraselmis chuii* extract, 100 mg of glucose, 50 mg of red ginseng extract, and 600 mg of starch were mixed and added with 100 mg of 30% ethanol to form granules, which were dried at 60°C and then filled in a pouch. The final weight of the contents was 1 g.

### [Formulation Example 7] Drink

150 mg of *Tetraselmis chuii* extract, 10 g of glucose, 50 mg of red ginseng extract, 2 g of citric acid, and 187.8 g of purified water were mixed and filled in a bottle. The final volume of the contents was 200 ml.

### [Formulation Example 8] Preparation of health food

1000 mg of *Tetraselmis chuii* extract
Vitamin mixture
70 µg of vitamin A acetate
1.0 mg of vitamin E
0.13 mg of vitamin B1
0.15 mg of vitamin B2
0.5 mg of vitamin B6
0.2 µg of vitamin B12
10 mg of vitamin C
10 µg of biotin
1.7 mg of nicotinamide
50 µg of folic acid
0.5 mg of calcium pantothenate
Mineral mixture
1.75 mg of ferrous sulfate
0.82 mg of zinc oxide
25.3 mg of magnesium carbonate
15 mg of potassium phosphate monobasic 55 mg of dicalcium phosphate
90 mg of potassium citrate
100 mg of calcium carbonate
24.8 mg of magnesium chloride

The mixing ratio of the vitamin and mineral mixtures was determined based on the ingredients relatively appropriate for health foods, but the mixing ratio may be modified arbitrarily. The ingredients may be mixed according to a conventional method for preparing health foods, granulated into granules, and used for the preparation of a composition for health foods according to a conventional method.

### [Formulation Example 9] Preparation of health beverage

1000 mg of *Tetraselmis chuii* extract
1000 mg of citric acid
100 g of oligosaccharide
2 g of plum concentrate
1 g of taurine
Purified water ad. 900 ml

The ingredients were mixed according to a conventional method for preparing health beverages. The resultant mixture was heated at 85°C with stirring for about 1 hour, filtered and put in a sterile 2L container. The mixture was then sealed and sterilized, and stored in a refrigerator until it would be used for preparation of the composition for health beverages of the present invention.

The ingredients relatively appropriate for beverages were mixed according to the preferred mixing ratio, but the mixing ratio may be adjusted according to regional and national preferences, such as demand class, demand country, and purpose of use.

It will be apparent to those skilled in the art that various changes and modifications may be made without departing from the scope of the invention as defined in the following claims.

While the present invention has been described with respect to the specific embodiments, it will be apparent to those skilled in the art that the above descriptions are only preferred embodiments and that the scope of the present invention is not limited thereto. Thus, the scope of the present invention should be defined by the appended claims

## Claims

1. An extract of *Tetraselmis* sp. microalgae for use in improving eye health,
wherein the extract is a hexane extract or a hexane fraction,
wherein the hexane extract is obtained using a solvent consisting of hexane, and
wherein the improvement of eye health is the prevention, amelioration or treatment of a retinal disease.

2. The extract of *Tetraselmis* sp. microalgae for use according to claim 1,
wherein the *Tetraselmis* sp. microalgae is *Tetraselmis chuii.*

3. The extract of *Tetraselmis* sp. microalgae for use according to claim 1 or claim 2,
wherein the extract is the hexane extract.

4. The extract of *Tetraselmis* sp. microalgae for use according to any one of claims 1 to 3,
wherein the retinal disease is at least one selected from the group consisting of retinopathy of prematurity, retinal degeneration, aged macular degeneration, diabetic retinopathy, hypertensive retinopathy, retinitis pigmentosa, Leber congenital amaurosis, retinal detachment, glaucoma, and optic neuropathy.

5. The extract of *Tetraselmis* sp. microalgae for use according to any one of claims 1 to 4
wherein the retinal disease is optic neuropathy caused by retinal optic nerve damage.

6. The extract of *Tetraselmis* sp. microalgae for use according to any one of claims to 5
wherein the improvement of eye health is the inhibition of retinal damage.

7. The extract of *Tetraselmis* sp. microalgae for use according to any one of claims 1 to 6,
wherein the improvement of eye health is the inhibition of retinal optic nerve damage.

## Patentansprüche

1. Extrakt aus *Tetraselmis*-sp.-Mikroalgen zur Verwendung für die Verbesserung der Augengesundheit,
wobei der Extrakt ein Hexanextrakt oder eine Hexanfraktion ist,
wobei der Hexanextrakt unter Verwendung eines aus Hexan bestehenden Lösungsmittels gewonnen wird, und
wobei die Verbesserung der Augengesundheit die Prävention, Besserung oder Behandlung einer Netzhauterkrankung ist.

2. Extrakt aus *Tetraselmis*-sp.-Mikroalgen zur Verwendung nach Anspruch 1, wobei die *Tetraselmis*-sp.-Mikroalgen *Tetraselmis chuii* sind.

3. Extrakt aus *Tetraselmis*-sp.-Mikroalgen zur Verwendung nach Anspruch 1 oder 2, wobei der Extrakt der Hexanextrakt ist.

4. Extrakt aus *Tetraselmis*-sp.-Mikroalgen zur Verwendung nach einem der Ansprüche 1 bis 3,
wobei die Netzhauterkrankung mindestens eine aus der Gruppe, bestehend aus Frühgeborenen-Retinopathie, Retinadegeneration, altersbedingte Makuladegeneration, diabetische Retinopathie, hypertensive Retinopathie, Retinitis pigmentosa, Lebersche kongenitale Amaurose, Netzhautablösung, Glaukom und Optikusneuropathie, ist.

5. Extrakt aus *Tetraselmis*-sp.-Mikroalgen zur Verwendung nach einem der Ansprüche 1 bis 4,
wobei die Netzhauterkrankung durch Schädigung des retinalen Sehnervs verursachte Optikusneuropathie ist.

6. Extrakt aus *Tetraselmis*-sp.-Mikroalgen zur Verwendung nach einem der Ansprüche 1 bis 5,
wobei die Verbesserung der Augengesundheit die Hemmung einer Netzhautschädigung ist.

7. Extrakt aus *Tetraselmis*-sp.-Mikroalgen zur Verwendung nach einem der Ansprüche 1 bis 6,
wobei die Verbesserung der Augengesundheit die Hemmung einer Schädigung des retinalen Sehnervs ist.

## Revendications

1. Extrait de microalgues *Tetraselmis* sp. destiné à être utilisé pour améliorer la santé oculaire,
dans lequel l'extrait est un extrait d'hexane ou une fraction d'hexane,
dans lequel l'extrait d'hexane est obtenu en utilisant un solvant constitué d'hexane, et
dans lequel l'amélioration de la santé oculaire est la prévention, l'atténuation ou le traitement d'une maladie rétinienne.

2. Extrait de microalgues *Tetraselmis* sp. destiné à être utilisé selon la revendication 1,
dans lequel les microalgues *Tetraselmis* sp. sont des *Tetraselmis chuii.*

3. Extrait de microalgues *Tetraselmis* sp. destiné à être utilisé selon la revendication 1 ou 2,
dans lequel l'extrait est l'extrait d'hexane.

4. Extrait de microalgues *Tetraselmis* sp. destiné à être utilisé selon l'une quelconque des revendications 1 à 3,
dans lequel la maladie rétinienne est au moins une maladie du groupe comprenant la rétinopathie du prématuré, la dégénérescence rétinienne, la dégénérescence maculaire liée à l'âge, la rétinopathie diabétique, la rétinopathie hypertensive, la rétinite pigmentaire, l'amaurose congénitale de Leber, le décollement de rétine, le glaucome et la neuropathie optique.

5. Extrait de microalgues *Tetraselmis* sp. destiné à être utilisé selon l'une quelconque des revendications 1 à 4,
dans lequel la maladie rétinienne est une neuropathie optique provoquée par une lésion du nerf optique rétinien.

6. Extrait de microalgues *Tetraselmis* sp. destiné à être utilisé selon l'une quelconque des revendications 1 à 5,
dans lequel l'amélioration de la santé oculaire est l'inhibition d'une lésion rétinienne.

7. Extrait de microalgues *Tetraselmis* sp. destiné à être utilisé selon l'une quelconque des revendications 1 à 6,
dans lequel l'amélioration de la santé oculaire est l'inhibition d'une lésion du nerf optique rétinien.
